Europäisches Patentamt

(19) European Patent Office

Office européen des brevets

(11) Numéro de publication: **0 115 723**
**B1**

(12) **FASCICULE DE BREVET EUROPEEN**

(45) Date de publication du fascicule du brevet:
01.04.87

(51) Int. Cl.⁴: **A 61 B 3/10**

(21) Numéro de dépôt: **83402455.6**

(22) Date de dépôt: **19.12.83**

(54) Dispositif de mesure de paramètres oculaires, notamment écart pupillaire, avec des réticules électro-optiques à commande numérique.

(30) Priorité: **22.12.82 FR 8221537**

(43) Date de publication de la demande:
**15.08.84 Bulletin 84/33**

(45) Mention de la délivrance du brevet:
**01.04.87 Bulletin 87/14**

(84) Etats contractants désignés:
**DE GB IT SE**

(56) Documents cités:
**FR - A - 1 506 352**
**FR - A - 2 390 751**

**ELECTRONIQUE & MICROELECTRONIQUE, no. 228, 15 novembre 1976, pages 39-51, Paris, FR., C. PERROT:
"Les cristaux liquides au service de la sécurité routière"**

(73) Titulaire: **ESSILOR INTERNATIONAL Cie Générale d'Optique, 1 Rue Thomas Edison Echat 902, F-94028 Creteil Cedex (FR)**

(72) Inventeur: **Cousyn, Bernard, 44 avenue de Paris, F-94300 Vincennes (FR)**
Inventeur: **Haddadi, Ahmed, 26 rue Brillet, F-94130 Nogent-Sur-Marne (FR)**
Inventeur: **Hennequin, Jean-Claude, 2 rue du Stade Congis-Sur-Therouanne, F-77440 Lizy-Sur-Ourcq (FR)**

(74) Mandataire: **CABINET BONNET-THIRION, 95 Boulevard Beaumarchais, F-75003 Paris (FR)**

## Description

L'invention a trait à un dispositif de mesure de paramètres oculaires d'un sujet, notamment écart pupillaire, en vue de l'adaptation de paires de lunettes.

Pour qu'une paire de lunettes soit convenablement adaptée au sujet qui la porte, il convient que les verres soient placés dans les anneaux de monture de sorte que leurs axes optiques principaux coïncident avec les axes optiques des yeux correspondants, dans des conditions moyennes d'utilisation. La monture fait office de référence secondaire de position pour les verres, sa position par rapport à la face du sujet étant définie essentiellement par l'appui de plaques nasales sur les côtés opposés de la racine du nez, et accessoirement par l'appui des branches sur les oreilles.

La position des verres par rapport à la monture se définit commodément en coordonnées cartésiennes avec un axe des abscisses horizontal (par référence à la face du sujet) et un axe des ordonnées vertical, par l'abscisse et l'ordonnée du centre optique du verre. L'origine des abscisses se situe naturellement dans le plan de symétrie vertical de la monture. Pour des raisons de commodité de finition des verres, on choisira généralement comme origine des ordonnées la droite horizontale qui passe par la partie inférieure des anneaux de monture, afin que l'ordonnée soit la distance entre le centre optique du verre et son bord inférieur.

On rappelle que l'écart pupillaire est la distance qui sépare les axes optiques des yeux lorsque le regard est à l'infini. La différence (vectorielle) entre les abscisses des centres optiques des verres doit correspondre à l'écart pupillaire du sujet.

Le brevet français FR-A 1 506 352, demandé le 4 Août 1966, décrit un dispositif perfectionné de mesure de l'écart pupillaire en déterminant pour chaque œil la position du reflet sur la cornée d'un point lumineux situé à l'infini, pour le regard fixé à l'infini. On obtient des déterminations analogues pour le regard fixé sur un point à distance finie, où convergent alors les axes optiques des yeux.

Le dispositif selon le brevet précité comprend essentiellement une boîte dans laquelle sont placées une lentille susceptible d'être déplacée parallèlement à elle-même suivant son axe optique principal, une source lumineuse au foyer de la lentille lorsque celle-ci est en position d'origine au plus loin de la source, celle-ci étant décalée géométriquement de l'axe optique par une lame semi-réfléchissante disposée à 45°, et sur deux côtés opposés de la boîte, perpendiculaires à l'axe optique de la lentille, respectivement un œilleton situé sur l'axe optique au foyer de la lentille en position origine et une façade comportant des plaques nasales et deux ouvertures pour simuler une monture de lunettes, les ouvertures encadrant l'emplacement destiné aux verres et étant munies de repères mobiles formant réticules. Pour les mesures on fait coïncider les repères mobiles et les reflets sur les cornées du point de source, tels qu'on peut les voir à travers l'œilleton. On notera que la position de la lentille définit pour le sujet une distance virtuelle de point de source, tandis que la visée de l'opérateur par l'œilleton s'effectue depuis un point en permanence en coïncidence optique avec le point source. La mesure de l'écart pupillaire pour un regard en convergence sur un point virtuel rapproché n'introduit pas d'erreur de parallaxe angulaire, et la position de réticule dans le plan des fenêtres, qui correspond au plan général des verres, définit la position souhaitable du centre optique des verres.

Ce dispositif dont la conception optique est excellente, souffre d'imperfections dans la partie mécanique de commande de déplacement des réticules. La précision souhaitable de quelques dixièmes de millimètres impose l'usage de crémaillères ou de vis micrométriques pour déplacer les index, ce qui conduit à des dispositifs fragiles, et de manœuvre lente. Les lectures de positions sont difficiles, avec des verniers ou des tambours associés à des échelles linéaires dès lors que la précision requise est supérieure au demi-millimètre. En outre les déplacements dans deux directions orthogonales de réticules viennent compliquer sérieusement les dispositions mécaniques de commande et d'affichage. En fait le dispositif décrit dans le brevet précité ne possède que des index à déplacement horizontal pour la détermination principalement des écarts pupillaires, et accessoirement de la distance de la monture de lunettes à l'œil, par visée de profil.

L'invention a pour objet un dispositif de mesure de paramètres oculaires capable de fournir rapidement des données précises pour l'adaptation de verres dans une monture de lunettes aux paramètres oculaires d'un sujet.

L'invention a également pour objet un tel dispositif qui fournisse, outre les écarts pupillaires, entre pupilles et entre chaque pupille et l'axe de symétrie de la monture, l'écart entre la pupille et le bord inférieur de la monture.

L'invention a encore pour objet un tel dispositif, qui fournit des données numériques directement exploitables.

A ces effets l'invention propose un dispositif de mesure de paramètres oculaires d'un sujet, notamment écart pupillaire, en vue de l'adaptation de paires de lunettes, dispositif comportant une façade avec des moyens de repérage par rapport à la face du sujet et des fenêtres correspondant à l'emplacement où se placent les verres, des moyens optiques avec un axe optique normal à la façade et centré sur celle-ci, ces moyens optiques comprenant une lentille convergente réglable en position sur l'axe optique à partir d'une origine proche de la façade, un ensemble d'un point source lumineux et d'un œilleton de visée, optiquement confondus, ensemble situé, du côté opposé à la façade par rapport à la lentille et au foyer de celle-ci lorsqu'elle est à sa position d'origine, et deux jeux de réticules disposés dans le plan de façade correspondantes avec des moyens de réglage couplés à des afficheurs de position, caractérisé en ce que lesdits jeux de réticules sont constitués chacun au moins d'une matrice diascopique à cristaux liquides et adressable en lignes et colonnes, lesdits moyens de réglage comprenant des générateurs numériques d'adresses.

Les matrices diascopiques à cristaux liquides sont sensiblement transparentes tant que les points, définis par leur adresse, ne reçoivent pas un signal de commutation. Lorsqu'un signal issu d'un générateur d'adresses est délivré à une ligne ou à une colonne, l'ensemble des points constituant la ligne ou la colonne intéressée est commuté, apparaissant comme un trait droit, horizontal ou vertical pour l'observateur. La position de la ligne ou de la colonne est en correspondance avec l'adresse numérique de commande, de sorte que cette adresse est un nombre représentatif de l'ordonnée ou de l'abscisse, dans le système de coordonnées de la façade, des traits formant réticule. On conçoit que la commande des générateurs de signaux numériques peut être rapide tout en restant précise, et que la multiplication des combinaisons de traits se résout en commutations de circuits, sans modifier la partie optique des réticules.

De préférence chaque jeu de réticules comporte deux matrices, une matrice supérieure centrée sur l'axe optique d'un œil de sujet moyen et une matrice inférieure centrée au niveau du bord inférieur de monture moyenne, et les moyens de réglage sont adaptés à délivrer des signaux d'adresse de lignes et de colonne à la matrice supérieure, et des signaux d'adresse de lignes seulement à la matrice inférieure.

On comprend que la matrice supérieure détermine l'abscisse (colonne) et l'ordonnée (ligne) du reflet du point de source sur la cornée, tandis que la matrice inférieure détermine l'ordonnée du bord inférieur d'anneau de monture. La combinaison des adresses de colones des deux matrices supérieures est représentative de l'écart pupillaire total, tandis que la combinaison des adresses de lignes des deux matrices d'un même jeu est représentative de la distance appropriée entre le centre optique et le bord inférieur du verre correspondant.

De préférence également les moyens de réglage sont adaptés à délivrer des signaux d'adresse, soit aux colonnes de matrices supérieures, soit aux lignes des deux matrices de chaque jeu. Cette disposition rend plus aisée la manœuvre du dispositif, en réduisant le nombre de réglages dans chaque mode de mesure. On notera que les réglages relatifs à la matrice supérieure sont perturbés si les yeux du sujet cessent temporairement de fixer l'image du point source. Par ailleurs la mesure de l'écart pupillaire s'effectue normalement lorsque le sujet ne porte pas la monture, étant donné que cette mesure est plus importante et requiert plus de précision que la mesure de position du bord de monture.

En disposition préférée les moyens d'affichage sont couplés à l'ensemble des moyens de réglage et adaptés à traduire la combinaison de signaux d'adresse en nombre représentatif de paramètres oculaires. Il est clair qu'à partir du moment où les signaux numériques délivrés par les générateurs d'adresse sont significatifs de coordonnées de points particuliers, il est aisé de déterminer par calcul numérique des données exploitables pour l'adaptation des verres sur une monture.

Les caractéristiques et avantages de l'invention ressortiront d'ailleurs de la description qui va suivre à titre d'exemple, en référence aux dessins annexés dans lesquels:

la fig. 1 est un schéma général d'un dispositif selon l'invention;

la fig. 2 est une vue plus détaillée d'une façade de dispositif selon l'invention;

les fig. 3A et 3B explicitent deux stades de mesures utilisant un dispositif selon l'invention;

la fig. 4 est un schéma de configuration des commandes électroniques d'un dispositif selon l'invention.

Selon la forme de réalisation choisie et représentée, un dispositif de mesure de paramètres oculaires comprend une boîte parallélépipédique 1, avec une façade 10 reproduisant le cadre d'une monture de lunettes, une lentille plan sphérique 11 dont l'axe optique principal est normal au plan de façade 10, et qui est réglable en position suivant cet axe optique principal, un œilleton de visée 12 disposé dans le côté de la boîte 1 opposé à la façade 10, centré sur l'axe optique de la lentille 11, la longueur de la boîte entre façade 10 et œilleton 12 étant telle que le foyer de la lentille 11 soit au centre de l'œilleton 12 lorsque cette lentille est dans sa position la plus rapprochée de la façade 10.

Un peu en avant de l'œilleton est disposée une lame 14, partiellement réfléchissante, orientée à 45° sensiblement de l'axe optique. Une source lumineuse 13 de faible étendue est disposée de telle sorte que son image par réflexion sur la lame 14, soit confondue avec le centre de l'œilleton 12.

La façade 10 comporte des éléments de repère de position de la tête du sujet dont les paramètres oculaires sont à déterminer. Ces éléments comprennent une paire de plaques nasales 19 et un appui pour le front, non représenté, qui serait situé juste au-dessus du bord supérieur de la façade 10. On comprend que les plaques nasales 19 forment repère de position en correspondance avec les plaques nasales d'une monture classique, tandis que l'appui pour le front définit la coïncidence entre le plan de plaque de façade et le plan moyen de face de monture, et accessoirement précise la mise en coïncidence du plan vertical de symétrie de la façade et le plan de symétrie de la tête du sujet.

La façade comporte des fenétres 15, 16, 17 et 18 constituées par des matrices à cristaux liquides, comme on le précisera plus en détail ci-après. Les fenêtres supérieures 15 et 16 sont carrées, avec un côté d'environ 16 millimètres, tandis que leurs centres sont écartés de la distance moyenne interpupillaire, soit environ 60 millimètres. Les fenêtres inférieures 17, 18 sont rectangulaires, avec une hauteur d'environ 27 millimètres et une largeur d'environ 50 millimètres, leurs centres étant écartés d'environ 70 millimètres entre eux, et distants de la ligne des centres des fenêtres supérieures 15, 16 d'environ 43 millimètres, ce qui est une valeur moyenne de la distance qui sépare le centre optique d'un verre de lunettes de son bord inférieur. On précise que les bords des fenêtres 15 à 18 sont verticaux et horizontaux, et que les fenêtres 15 et 17 sont symétriques des fenêtres 16 et 18 respectivement par rapport à la droite médiatrice verticale de la façade 10.

Les matrices à cristaux liquides comportent des cellules sensiblement carrées, disposées en lignes, horizontales, et en colonnes, verticales, en sorte qu'il soit possible de commuter chaque point de matrice par des signaux de commande adressés à la ligne et à la colonne dont l'intersection est constituée par la cellule considérée.

Néanmoins, dans le dispositif de mesure de paramètres oculaires, les signaux de commande sont adaptés à commuter toutes les cellules d'une ligne ou d'une colonne simultanément. Pratiquement si la commutation d'une cellule est commandée par un niveau logique 1, au lieu d'appliquer un niveau $+\frac{1}{2}$ sur la ligne et $-\frac{1}{2}$ sur la colonne d'adresse, on applique un niveau 1 sur la ligne ou la colonne qui doit commuter.

Les circuits de commande comprennent une unité logique 20, couplée sélectivement aux entrées de lignes et de colonnes des matrices des fenêtres 15 et 16, et aux entrées de lignes des matrices des fenêtres 17 et 18. Un clavier de commande 21 constitue, conjointement avec l'unité logique 20 à laquelle il est couplé, un générateur d'adresses de commande de commutation.

Chaque cellule de cristaux liquides comprend une lame de liquide nématique biréfringent entre deux électrodes transparentes. Deux lames polarisantes convenablement orientées sont disposées de part et d'autre de la matrice. Lorsque la cellule n'est pas commutée par application d'un signal de commande la rotation du plan de polarisation induite par le cristal liquide nématique sur la lumière entre les lames polarisantes autorise le passage de la lumière à travers la matrice; par contre la commutation de la cellule annule la rotation du plan de polarisation entre les deux lames polarisantes. En observation par transparence, ou diascopique, une cellule commutée apparaît opaque, et une cellule non commutée transparente. Dans l'application au dispositif de mesure de paramètres oculaires décrit ici, où toutes les cellules d'une ligne ou d'une colonne sont commutées simultanément, la ligne ou la colonne apparaît comme un trait opaque contrastant avec le reste de la matrice qui reste transparent. On précisera plus loin le processus de commande de commutation.

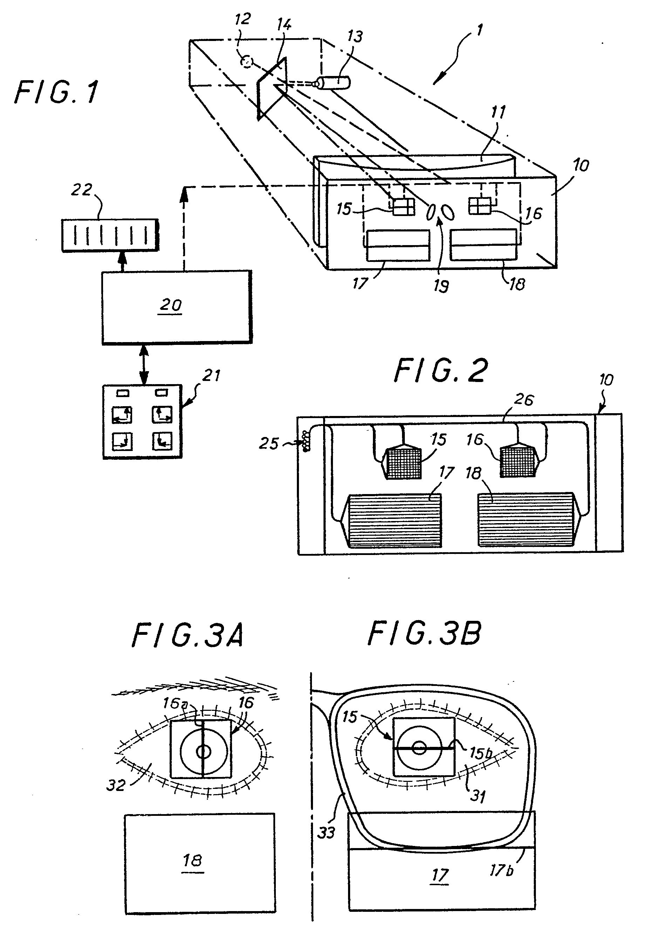

La figure 2 présente une vue plus détaillée de la constitution électro-optique de la façade 10. D'un connecteur 25 part un faisceau de conducteurs 26, qui aboutissent aux commandes en lignes ou en colonnes des matrices des fenêtres supérieures 15 et 16, et aux commandes en ligne des matrices des fenêtres 17 et 18. Pour des raisons qui seront développées plus loin, dans un premier mode de fonctionnement de l'unité logique 20 (figure 1), les matrices des fenêtres supérieures 15 et 16 reçoivent chacune un signal de commutation d'une colonne, avec un adresse déterminée par le clavier 21, tandis que dans un second mode de fonctionnement les matrices des quatre fenêtres 15-18 reçoivent chacune un signal de commutation d'une ligne, avec une adresse également déterminée, indépendamment pour chaque matrice, par le clavier 21. Dans le premier mode il apparaît un trait vertical sombre dans les fenêtres supérieures 15 et 16, réglable en position, et dans le second mode il apparaît dans chaque fenêtre un trait horizontal sombre, réglable en position.

On a compris que, comme avec un dispositif classique de mesure de l'écart pupillaire, le sujet, à qui doit être adaptée une paire de lunettes, place sa face contre la façade 10, de sorte que ses pupilles se trouvent en regard des fenêtres supérieures 15 et 16. La source 13, vue par réflexion sur la lame 14, et par réfraction à travers la lentille 11, en position d'origine près de la façade 10, apparaît comme un point à l'infini pour le sujet, à hauteur d'horizon. Les axes de visée des yeux du sujet sont donc parallèles, en direction moyenne, c'est-à-dire en vision de face à hauteur d'horizon. Pour l'opérateur qui observe à travers l'œilleton 12, les yeux du sujet apparaissent comme dans une loupe de faible puissance, avec un point lumineux au centre de la cornée, point formé par le reflet de la source 13 sur le miroir convexe de la cornée. En outre apparaissent en superposition les traits sombres correspondant aux colonnes ou lignes commutées des matrices des fenêtres supérieures 15 et 16.

Les figures 3A et 3B illustrent schématiquement les deux modes de mesure, représentés par commodité l'un pour l'œil droit, l'autre pour l'œil gauche; on aura compris que chaque mode s'applique aux deux yeux.

Le premier mode, figure 3A, est destiné à la mesure de l'écart pupillaire; seules les matrices des fenêtres 15 et 16 reçoivent des signaux, et adressés en colonne, de sorte qu'il apparaît un trait sombre vertical 16a. Le réglage des signaux d'adresse par le clavier 21 (figure 1) est effectué en sorte que le trait 16a passe par le reflet de la source lumineuse sur la cornée. Chaque signal d'adresse correspond alors à la distance du centre de pupille de l'œil correspondant à l'axe vertical de symétrie de la façade, et la somme de ces distances à l'écart interpupillaire. En réponse à cette mesure l'unité logique 20 (figure 1), qui possède en mémoire des paramètres de dimension et de position dans la façade de la matrice, affiche l'écart droit, l'écart gauche et l'écart total.

Le second mode de mesure, figure 3B, est destiné à la détermination de la position du centre optique du verre dans la monture; les matrices des quatre fenêtres 15–18 reçoivent des signaux d'adresse en lignes, ce qui se traduit par l'apparition de traits sombres horizontaux (15b dans la fenêtre 15, 17b dans la fenêtre 17). La mesure est effectuée avec la monture en place sur la face du sujet; il est clair que les plaques nasales 19 de la façade 10, représentée en figure 1, sont remplacées par des drageoirs appropriés, qui assurent que la monture 33 se place convenablement sur la façade. Par manœuvre de clavier 21 (figure 1), on règle les signaux d'adresse de sorte que le trait 15b et le trait 16b (non représenté) passent par le centre de la pupille de l'œil 31 correspondant; le trait 17b et le trait 18b non représenté, sont ajustés en position pour passer par le bord inférieur de la monture. La combinaison des signaux, à droite et à gauche, compte tenu des paramètres de dimensions des matrices et de leurs positions dans la façade, permet l'affichage de la distance à prévoir entre le centre optique et le bord inférieur du verre pour la mise en place sur la monture.

Dans la façade 10 représentée à la figure 2, les matrices des fenêtres supérieures 15 et 16 présentent soixante-quatre lignes et soixante-quatre colonnes dans un carré de 15,75 mm de côté, et ont leur bord interne à 22 mm de l'axe vertical de symétrie de la façade. Les points de matrice mesurent 0,125 × 0,125 mm et sont espacés de 0,125 m. Les matrices des fenêtres inférieures 17 et 18 mesurent 27 × 50 mm et comportent 55 lignes de largeur 0,3 espacées de 0,2 mm. Les bords internes de ces matrices 17, 18 sont distants de 10 mm des bords inférieurs des matrices des fenêtres supérieures 15 et 16.

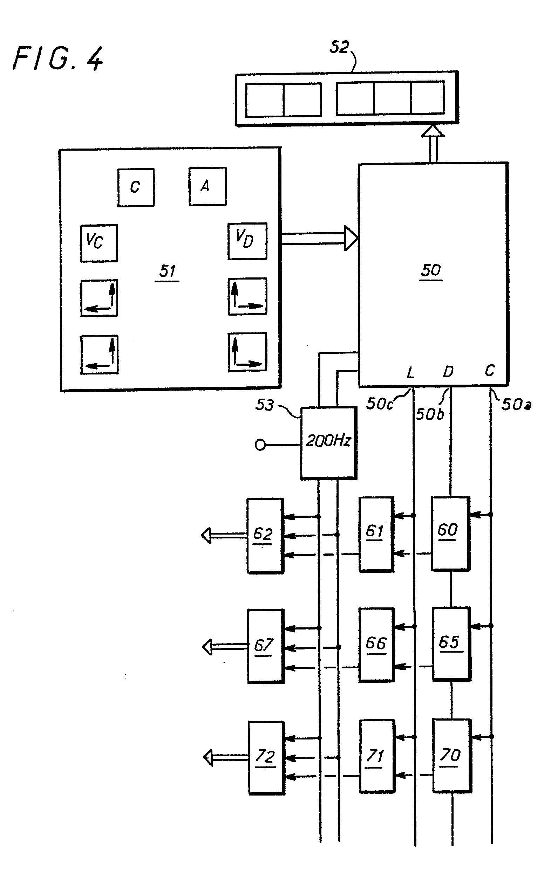

La configuration des circuits de commande des matrices est représentée figure 4. Le cœur de la commande est constitué par un microprocesseur 50, couplé à un clavier de comande 51, un afficheur numérique 52, et des dispositifs d'interface pour la commande directe des matrices. Le microprocesseur 50 comporte une horloge interne à 2 MHz, et est programmé de telle façon que les modes de mesure se succèdent, le second débutant lorsque le premier est achevé. Le clavier 51 porte des touches de croissance et de décroissance d'adresse, surmonté d'une touche de validation, en colonnes pour l'œil droit et l'œil gauche. L'action sur les touches de croissance ou de décroissance de signal d'adresse provoquent des déplacements des traits sombres dans les fenêtres. Lorsque le pointage est correct l'appui sur la touche validation introduit les données dans le microprocesseur pour le calcul des valeurs à afficher. On comprend que tout ceci est obtenu par le logiciel de programmation du microprocesseur.

Pour la formation des signaux d'adresse, on a affecté des adresses successives 1 à 64 aux lignes de matrice supérieure gauche, 65 à 128 aux colonnes de cette même matrice, 129 à 192 aux lignes de la matrice supérieure droite, 193 à 256 aux colonnes de cette matrice, 257 à 311 aux lignes de la matrice inférieure droite, et 312 à 366 aux lignes de la matrice inférieure gauche. En début de cycle de fonctionnement du microprocesseur, des signaux d'horloge sur la sortie 50a du microprocesseur attaquent une succession de registres à décalage 60, 65, 70, d'une capacité de 32 cellules chacun, tandis que des signaux apparaissent sur la sortie 50b simultanément aux impulsions d'horloge dont le rang correspond aux adresses des lignes ou colonnes à commuter des matrices. A la 366e impulsion d'horloge, le chargement du registre à décalage 60, 65, 70 . . ., est achevé et un signal de chargement apparaît sur la sortie 50c du microprocesseur 50, en synchronisme avec la 367e impulsion d'horloge. Les circuits de chargement 61, 66, 71 . . ., disposés entre les éléments 60, 65, 70 . . ., de registre à décalage et les pilotes de commutation 62, 67, 72 . . ., couplés aux lignes et colonnes des matrices à cristaux liquides, transfèrent le contenu des éléments de registre 60, 65, 70 . . ., aux pilotes 62, 67, 72 . . . Les circuits pilotes 62, 67, 72 . . . ont une capacite de 32 lignes ou colonnes, mais ceux qui sont couplés aux lignes des matrices inférieures comportent seulement 22 ou 23 sorties occupées. Les circuits pilotes reçoivent un signal de polarisation à 200 Hz formé par le générateur 53, couplé au microprocesseur 50, signal qui sera transmis aux lignes et colonnes suivant le signal d'adresse transmis par les ciruits de chargement. Le cycle complet de microprocesseur dure de 30 à 50 ms, qui correspond à un fonctionnement correct des cristaux liquides, compte tenu de leurs inerties de commutation. Mais le chargement des registres à décalage, pendant lequel le microprocesseur est affecté à la génération d'adresses, dure seulement 183 microsecondes.

Les registres à décalage 60, 65, 70, les circuits de chargement 61, 66, 71 et les pilotes 62, 67, 72 sont des circuits intégrés disponibles sur le marché et utilisés classiquement, pour la commutation de cristaux liquides notamment. Il n'est donc pas nécessaire de s'étendre sur leur fonctionnement propre.

Il est évident que les résultats de mesure indiqués par l'afficheur 52 peuvent être transmis à une imprimante, pour imprimer une fiche pour l'usinage de finition des verres pour les ajuster à la monture.

On notera que l'expression «fenêtre», qui suggère en soi une ouverture destinée au passage de lumière dans une paroi, ne doit pas être prise, dans la présente description dans un sens trop étroit, où les parties de face autres que les fenêtres seraient opaques. Selon l'invention ces parties peuvent être aussi bien transparentes qu'opaques.

Il est non moins évident que les matrices inférieures peuvent être constituées avec une seule colonne, les lignes s'étendant en réseau sur toute la largeur de la fenêtre.

Il est également clair que les matrices à cristaux liquides peuvent être réalisées avec une commande point par point, chaque point possédant une entrée de commande individuelle. Lorsque cela sera le cas, on leur associera des interfaces de commande capables, en réponse à des signaux d'adresse en lignes et colonnes, d'émettre des signaux de commande individuelle vers les points voulus.

## Revendications

1. Dispositif de mesure de paramètres oculaires d'un sujet, notamment écart pupillaire, en vue de l'adaptation de paires de lunettes, dispositif comportant une façade (10) avec des moyens de repérage (19) par rapport à la face du sujet et des fenêtres (15, 16; 17, 18) correspondant à l'emplacement où se placent les veres, des moyens optiques (11–14) avec un axe optique normal à la façade (10) et centré sur celle-ci, ces moyens optiques comprenant une lentille (11) convergente réglable en position sur l'áxe optique à partir d'une origine proche de la façade (10), un ensemble d'un point source lumineuse (13) et d'un œilleton (12) de visée, optiquement confondus, ensemble situé, du côté opposé à la façade (10) par rapport à la lentille (11) et au foyer de celle-ci lorsqu'elle est à sa position d'origine, et deux jeux de réticules disposés dans le plan de façade et réglables en position dans l'encadrement des fenêtres (15, 16; 17, 18) correspondantes avec des moyens de réglage couplés à des afficheurs de position (22), caractérisé en ce que lesdits jeux de réticules sont constitués chacun au moins d'une matrice diascopique à cristaux liquides et adressables en lignes et colonnes, lesdits moyens de réglage comprenant des générateurs numériques (20, 21) d'adresses.

2. Dispositif selon la revendication 1, caractérisé en ce que chaque jeu de réticules comporte deux matrices, une matrice supérieure (15, 16) centrée sur l'axe optique d'un œil de sujet moyen, et une matrice inférieure (17, 18) centrée au niveau du bord inférieur de monture moyenne, et en ce que les moyens de réglage (20, 21, 50, 51) sont adaptés à délivrer des signaux d'adresse de lignes et de colonnes à la matrice supérieure (15, 16) et des signaux d'adresse de lignes à la matrice inférieure (17, 18).

3. Dispositif selon la revendication 2, caractérisé en ce que les moyens de réglage (20, 21, 50, 51) sont adaptés, soit à délivrer des signaux d'adresse de colonnes à la matrice supérieure (15, 16), soit des signaux d'adresse de lignes à la matrice supérieure (15, 16) et à la matrice inférieure (17, 18).

4. Dispositif selon une quelconque des revendications 1 à 3, caractérisé en ce que lesdits moyens d'affichage (22, 52) sont couplés à l'ensemble desdits moyens de réglage (20, 21, 50, 51) et adaptés à traduire des combinaisons de signaux d'adresse en nombres représentatifs de paramètres oculaires.

## Patentansprüche

1. Messvorrichtung für Augenparameter einer Versuchsperson, insbesondere des Pupillenabstandes zur Anpassung einer Brille, mit einer Kopfplatte (10) mit Markierungselementen (19) für das Gesicht der Versuchsperson, und mit Fenstern (15, 16, 17, 18) an den Stellen, wo die Gläser angeordnet werden, mit einer optischen Einrichtung (11–14) mit einer optischen Achse senkrecht zu der Kopfplatte (10) und zentriert zu dieser, wobei die optische Einrichtung eine Konvexlinse (11) aufweist, die auf der optischen Achse ausgehend von einer Ausgangsstellung in Nähe der Kopfplatte (10) einstellbar ist, mit einer Anordnung aus einer punktförmigen Lichtquelle (13) und einer Zielokularblende (12), die optisch verbunden sind, wobei die Anordnung in Bezug zu der Linse (11) auf der Seite gegenüber der Kopfplatte (10) und im Brennpunkt der Linse angeordnet ist, wenn diese in ihrer Ausgangsstellung ist, und mit zwei Sätzen von Fadenkreuzen, die in der Ebene der Kopfplatte angeordnet und in den Umrahmungen der entsprechenden Fenster über Einstellmittel verstellbar sind, die mit Positionsanzeigegeräten (22) gekoppelt sind, dadurch gekennzeichnet, dass die Sätze der Fadenkreuze jeweilig aus wenigstens einer diaskopischen Matrix aus Flüssigkristallen bestehen, die in Linien und Spalten adressierbar sind, und dass die Regeleinrichtung numerische Adressengeneratoren (20, 21) aufweist.

2. Vorrichtung nach Anspruch 1, dadurch gekennzeichnet, dass jeder Fadenkreuzsatz zwei Matrizen aufweist, dass eine obere Matrix (15, 16) auf der optischen Achse eines Auges der mittleren Versuchsperson und eine untere Matrix (17, 18) in Höhe des unteren Randes des mittleren Brillengestelles zentriert ist, und dass die Regeleinrichtung (20, 21, 50, 51) Adressignale der Linien und Spalten an die obere Matrix (15, 16) und Adressignale der Linien an die untere Matrix (17, 18) liefert.

3. Vorrichtung nach Anspruch 2, dadurch gekennzeichnet, dass die Regeleinrichtung (20, 21, 50, 51) entweder Adressignale der Spalten an die obere Matrix (15, 16) oder Adressignale der Linien an die obere Matrix (15, 16) und an die untere Matrix (17, 18) liefert.

4. Vorrichtung nach einem der Ansprüche 1 bis 3, dadurch gekennzeichnet, dass die Anzeigeeinrichtung (22, 52) mit der Anordnung der Regeleinrichtung (20, 21, 51, 51) gekoppelt ist und die Kombinationen der Adressignale in Zahlen überträgt, die repräsentativ sind für Augenparameter.

## Claims

1. Device for measuring ocular parameters of a subject, especially the spacing between pupils, with a view to the adaptation of pairs of spectacles, the device comprising a front (10) with means for registration (19) relative to the face of the subject and windows (15, 16; 17, 18) corresponding to the position where the lenses are located, optical means (11–14) with an optical axis normal to the front (10) and centred on the latter, said optical means comprising a converging lens (11) regulable in position on the optical axis from an origin near the front (10), an assembly of a point light source (13) and of an eyepiece diaphragm (12), optically coincident, the assembly being situated on the side opposite the front (10) relative to the lens (11) and at the focus of the latter when it is in its origin position and two sets of reticles disposed in the plane of the front and regulable in position within the frame of the corresponding windows (15, 16, 17, 18) by regulating means coupled to position indicators (22), characterised in that the said sets of reticles each comprise at least one liquid crystal diascopic matrix and are addressable in lines and columns, the said regulating means comprising digital generators (20, 21) of addresses.

2. Device according to Claim 1, characterised in that each set of reticles comprises two matrices, an upper matrix (15, 16) centred on the optical axis of an eye of an average subject, and a lower matrix (17, 18) centred at the level of the lower edge of an average frame, and in that the regulating means (20, 21, 50, 51) are adapted to supply line and column address signals to the upper matrix (15, 16) and line address signals to the lower matrix (17, 18).

3. Device according to Claim 2, characterised in that the regulating means (20, 21, 50, 51) are adapted to supply either column address signals to the upper matrix (15, 16) or line address signals to the upper matrix (15, 16) and to the lower matrix (17, 18).

4. Device according to any one of claims 1 to 3, characterised in that the said indicating means (22, 52) are coupled to all the said regulating means (20, 21, 50, 51) and adapted to convert combinations of address signals into numbers representing ocular parameters.

FIG.1

FIG.2

FIG.3A

FIG.3B

# FIG. 4